Europäisches Patentamt

European Patent Office

Office européen des brevets

(11) Publication number: **0 411 206 B1**

## EUROPEAN PATENT SPECIFICATION

(45) Date of publication of patent specification: **25.08.93**  (51) Int. Cl.⁵: **A61L  9/01**

(21) Application number: **89119783.2**

(22) Date of filing: **25.10.89**

The file contains technical information submitted after the application was filed and not included in this specification

(54) **Deodorant and deodorizing method.**

(30) Priority: **02.08.89 JP 199469/89**

(43) Date of publication of application:
**06.02.91 Bulletin  91/06**

(45) Publication of the grant of the patent:
**25.08.93 Bulletin  93/34**

(84) Designated Contracting States:
**DE FR GB IT**

(56) References cited:
**EP-A- 0 037 944**
**EP-A- 0 128 891**
**EP-A- 0 261 422**
**EP-A- 0 282 287**

**CHEMICAL ABSTRACTS, vol. 109, no. 20, 14th November 1988, page 332, abstract no. 175631c, Columbus, Ohio, US; & JP-A-63 130 136 (NIPPON KAYAKU CO., LTD) 02-06-1988**

(73) Proprietor: **Nippon Kayaku Kabushiki Kaisha**
**11-2, Fujimi-cho 1 chome Chiyoda-ku Tokyo(JP)**

(72) Inventor: **Yoshida, Norikazu**
**239 Iwahana-machi**
**Takasaki-shi Gunma-ken(JP)**
Inventor: **Matsumoto, Mutsumi**
**1-23-14 Nakai-machi**
**Takasaki-shi Gunma-ken(JP)**

(74) Representative: **Türk, Gille, Hrabal, Leifert**
**Brucknerstrasse 20**
**D-40593 Düsseldorf (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3. 10/3.6/3.3. 1)

## Description

The present invention relates to a deodorant which can be adapted to strong offensive odors and can be widely utilized in from housing to industrial fields, and a deodorizing method using the deodorant.

There are various sources of offensive odors, such as garbage, refrigerators, lavatories, manufacturing plants, livestock ranches, sewage disposing plants, etc. In addition, many places such as hospitals, hotels and restaurants have odors characteristic thereof which may not be said to be offensive odors. As the substances which generates such offensive odors, ammonia, mercaptans, sulfides, amines, acetaldehyde, etc. are known, but actually offensive odors are more complicated and the offensive ingredients thereof are not restricted to the above-described ones. With the recently increasing demand for the technique for removing these offensive odors, the extensive study of such technique has been undergone. For example, the following methods are proposed.

(1) A method to mask offensive odors by the fragrance of aromatic substances.

(2) An adsorbing method to adsorb the substances causing offensive odors by using an adsorbent such as active carbon.

(3) Acid-base neutralizing method to remove offensive odors by neutralizing offensive-odor substances with acids or bases.

(4) Chemical oxidation-reduction method to chemically decompose offensive-odor substances.

Each of the above-described methods have serious defects. For example, the masking method cannot be said to be a substantial method. In the adsorbing method, since the adsorbing amount is limited according to saturated amount of adsorption, it cannot be adapted to a strong offensive odor. The acid-base neutralizing method is applicable only to substances which can be neutralized. The chemical decomposing method is considered to be the most promising technique, but no method has a sufficient deodorizing activity in the present state of art.

Accordingly, it is an object of the present invention to solve the above-described problems in the prior art and to provide a deodorant which has a wide applicability, a strong deodorizing activity and a long life.

One of the present inventors invented a deodorant comprising at least one selected from the group consisting of phosphoric acid, phosphorous acid, hypophosphorous acid and the salts thereof and a peroxide as the main ingredients and, if necessary, containing a metal oxide, a metal salt and the like, and preferably carried by a porous carrier, and filed the patent application (EP-A-0261422). This deodorant has a high deodorizing activity against various offensive-odor substances irrespective of acidic or basic, and is more stable and more active than any of the conventional deodorants, and it has been proposed as a deodorant which can solve the problems of the conventional deodorants. However, as a result of continuous investigation carried out by the present inventors so as to find out a deodorant having an enhanced deodorizing activity, it has been found that a composition produced by adding a specific metal oxide to calcium peroxide and/or sodium percarbonate and carrying the mixture on active carbon having a surface pH of not less than 7 is a more active deodorant. The present invention has been achieved on the basis of this finding.

In a first aspect of the present invention, there is provided a deodorant comprising calcium peroxide and/or sodium percarbonate, at least one oxide selected from the group consisting of the oxides of titanium, manganese, iron, magnesium, aluminum and silicon, and active carbon having a surface pH of not less than 7.

In a second aspect of the present invention, there is provided a method for deodorizing a gas comprising offensive-odor substances, which comprises bringing the gas into contact with the deodorant of the present invention.

In a third aspect of the present invention, there is provided a method for deodorizing an odor-generating source, which comprises adding the deodorant of the present invention to the source.

Active carbon used in the present invention may be any active carbon that has a surface pH of not less than 7, preferably not less than 7.5. The use of active carbon having a large specific surface area such as not less than 600 $m^2/g$, more preferably not less than 800 $m^2/g$ can enhance the deodorizing activity.

As the property of active carbon as an adsorbent, a large specific surface area is generally noticed. However, in the specific composition of the present invention, the deodorizing activity is different depending upon the surface pH thereof, and when active carbon having a surface pH of not less than 7 is used, the deodorizing activity is remarkably enhanced.

The "surface pH" here means the pH of liquid obtained by boiling a mixture of 1 g of a sample (active carbon) and 100 mℓ of water added thereto for 5 minutes, cooling the mixture to ordinary temperature and adding water until the total volume of the mixture becomes 100 mℓ, as standardized by Japanese Industrial Standard JIS K1470.

2

The content of active carbon in the deodorant composition is not restricted, but preferably 10 to 95 wt%, more preferably 30 to 90 wt%.

The content of at least one oxide selected from the group consisting of the oxides of titanium, manganese, iron, magnesium, aluminum and silicon in the deodorant composition is preferably 0.5 to 80 wt%, more preferably 2 to 60 wt%, and the ratio of the oxide to calcium peroxide and/or sodium percarbonate is not restricted, but preferably 1 : 0.1 to 10, more preferably 1 : 0.2 to 5 by weight.

The process for preparing the deodorant of the present invention is not restricted, but a method of uniformly mixing ingredients in the form of powder is simple and preferable.

The deodorant of the present invention has a more excellent deodorizing activity than a conventional one, and improved anti-hygroscopic property and forming property even if one selected from the group consisting of phosphoric acid, phosphorous acid, hypophosphorous acid and the salts thereof is not contained therein. However, the deodorant of the present invention may further contain an acidic ingredient such as phosphoric acid and sulfuric acid.

It is observed that the addition of the acidic ingredient sometimes slightly enhances the deodorizing activity in treating basic offensive-odor substances.

The method of using the deodorant of the present invention is not restricted. For example, it may be formed into granules or pellets and be put in a permeable bag or container for use in refrigerators, lavatories, living rooms, or the inside of vehicles,. It is possible to forcibly pass gas having offensive odors which is emitted from sewage disposing plants, manufacturing plants, incinerating plants, livestock ranches, etc. through the layer of the deodorant of the present invention. It is also possible to apply or mix the deodorant of the present invention to or with packaging materials, and interior decorating materials such as wall paper. It is further possible to add the deodorant of the present invention directly to the source of offensive odors such as kitchen garbage, sludge, excreta of livestock, etc.

The deodorant of the present invention is capable of quickly decomposing offensive-odor substances. In addition, the deodorant of the present invention is applicable for wide purposes because it is usable irrespective of acidic substance or basic substance.

The present invention will be explained more precisely with reference to the following non-limitative examples.

## Example 1

20 parts by weight of titanium oxide, 10 parts by weight of calcium peroxide and 70 parts by weight of active carbon having a surface pH of 10.3 and a specific surface area of 1100 $m^2$/g were uniformly mixed in the form of a powder to obtain a deodorant composition. In a polyethylene container, 1 g of the thus-obtained composition was placed and 500 m$\ell$ of a sample gas containing 1000 ppm of methyl mercaptan, 500 ppm of hydrogen sulfide and 500 ppm of ammonia which had been prepared in advance was introduced thereinto. The container was sealed and the change in concentration was measured for each component. The results are shown in Table 1.

## Examples 2 to 7

The deodorant compositions having the respective ingredients shown in Table 1 were obtained in the same way as in Example 1. The same active carbon as in Example 1 is used in any of these Examples. The deodorizing effect of each composition was measured in the same way as in Example 1. The results are shown in Table 1.

## Example 8

The deodorants were produced in the same way as in Example 1 except that various kinds of active carbon having different surface pH were used. The results are shown in Table 2. As is apparent from the results, there is significant difference in deodorizing activity between a deodorant containing active carbon having a pH of less than 7 and a deodorant containing active carbon having a pH of not less than 7.

Table 1

| Example | Composition (weight ratio 20/10/70) | Offensive-odor substance | Concentration (ppm) | | |
|---|---|---|---|---|---|
| | | | 10 min. after | 20 min. after | 30 min. after |
| 1 | $TiO_2$/$CaO_2$/active carbon | Methyl mercaptan Hydrogen sulfide Ammonia | 8 3 15 | 1 0 5 | 0 0 0 |
| 2 | $MnO_2$/$CaO_2$/active carbon | Methyl mercaptan Hydrogen sulfide Ammonia | 5 2 10 | 0 0 0 | 0 0 0 |
| 3 | $Fe_2O_3$/$CaO_2$/active carbon | Methyl mercaptan Hydrogen sulfide Ammonia | 11 4 18 | 3 1 5 | 0 0 0 |
| 4 | MgO/$CaO_2$/active carbon | Methyl mercaptan Hydrogen sulfide Ammonia | 10 5 12 | 2 1 6 | 0 0 0 |
| 5 | $Al_2O_3$/$CaO_2$/active carbon | Methyl mercaptan Hydrogen sulfide Ammonia | 11 4 20 | 2 1 8 | 0 0 0 |
| 6 | $SiO_2$/$CaO_2$/active carbon | Methyl mercaptan Hydrogen sulfide Ammonia | 12 5 22 | 2 1 7 | 0 0 0 |
| 7 | $TiO_2$/$Na_2C_2O_6$/active carbon | Methyl mercaptan Hydrogen sulfide Ammonia | 9 4 15 | 2 0 5 | 0 0 0 |

Table 2

| pH of active carbon | Offensive-odor substance | Concentration (ppm) | | |
|---|---|---|---|---|
| | | 10 min. after | 20 min. after | 30 min. after |
| 12.2 | Methyl mercaptan<br>Hydrogen sulfide<br>Ammonia | 9<br>4<br>17 | 2<br>0<br>7 | 0<br>0<br>0 |
| 10.3 | Methyl mercaptan<br>Hydrogen sulfide<br>Ammonia | 8<br>3<br>15 | 1<br>0<br>5 | 0<br>0<br>0 |
| 8.0 | Methyl mercaptan<br>Hydrogen sulfide<br>Ammonia | 10<br>6<br>14 | 2<br>0<br>6 | 0<br>0<br>0 |
| 6.0 | Methyl mercaptan<br>Hydrogen sulfide<br>Ammonia | 22<br>12<br>30 | 11<br>7<br>14 | 2<br>2<br>2 |
| 3.8 | Methyl mercaptan<br>Hydrogen sulfide<br>Ammonia | 28<br>14<br>20 | 15<br>8<br>9 | 4<br>2<br>0 |

Examples 9 to 13

The deodorants were produced in the same way as in Examples 2 to 6 except for using sodium percarbonate in place of calcium peroxide and the deodorizing effect of each deodorant was measured in the same way as in Example 1. The results were almost the same as those in Examples 2 to 6, respectively.

Example 14

Into a 2 litre-glass container, was placed 100 g of dehydrated cake (water content: 80%) of the sludge used in sewage treatment and 1 g of the deodorant obtained in Example 1 was added thereto. The container was sealed and allowed to stand at 30°C. After 24 hours, the concentrations of hydrogen sulfide and methyl mercaptan were measured. As a result, it was observed that the concentrations of hydrogen sulfide and methyl mercaptan were respectively 350 ppm and 220 ppm when the deodorant was not used, whereas, not more than 5 ppm respectively when the deodorant was used.

Examples 15 to 20

The concentrations of hydrogen sulfide and methyl mercaptan were measured in the same way as in Example 14 except for using the deodorants obtained in Examples 2 to 7, respectively. In each case, the concentrations of both of hydrogen sulfide and methyl mercaptan were not more than 5 ppm.

**Claims**

1. A deodorant comprising (A) at least one oxide selected from the group consisting of the oxides of titanium, manganese, iron, magnesium, aluminum and silicon,(B) calcium peroxide, sodium percarbonate or a mixture thereof, and (C) active carbon having a surface pH of not less than 7.

2. A deodorant according to claim 1, wherein (A) is titanium oxide, and (B) is calcium peroxide.

3. A deodorant according to either of claims 1 and 2, wherein the ratio of (A) : (B) is 1 : 0.1 to 10 by weight ratio and the ratio of active carbon in said deodorant is 10 to 95 wt%.

4. A deodorant according to claim 1 or 2, wherein the specific surface area of active carbon is not less than 600 $m^2$/g.

5. Use of the composition of any of Claims 1-4 as a deodorant.

**Patentansprüche**

1. Deodorant, das umfaßt
   A) mindestens ein Oxid, ausgewählt aus der Gruppe, die besteht aus den Oxiden von Titan, Mangan, Eisen, Magnesium, Aluminium und Silicium,
   B) Calciumperoxid, Natriumpercarbonat oder eine Mischung davon und
   C) Aktivkohle mit einem Oberflächen-pH-Wert von nicht weniger als 7.

2. Deodorant nach Anspruch 1, worin
   A) Titanoxid ist und
   B) Calciumperoxid ist.

3. Deodorant nach einem der Ansprüche 1 und 2, worin das Gewichtsverhältnis (A):(B) 1:0,1 bis 10 beträgt und der Mengenanteil der Aktivkohle in dem Deodorant 10 bis 95 Gew.-% beträgt.

4. Deodorant nach Anspruch 1 oder 2, worin die spezifische Oberflächengröße der Aktivkohle nicht weniger als 600 $m^2$/g beträgt.

5. Verwendung der Zusammensetzung nach einem der Ansprüche 1 bis 4 als Deodorant.

**Revendications**

1. Désodorisant comprenant (A) au moins un oxyde choisi dans le groupe formé par les oxydes de titane, de manganèse, de fer, de magnésium, d'aluminium et de silicium, (B) du peroxyde de calcium, du percarbonate de sodium ou un mélange d'entre eux, et (C) du charbon actif ayant un pH superficiel d'au moins 7.

EP 0 411 206 B1

2. Désodorisant selon la revendication 1, dans lequel (A) est l'oxyde de titane et (B) est le peroxyde de calcium.

3. Désodorisant selon l'une ou l'autre des revendications 1 et 2, dans lequel le rapport (A):(B) est de 1 : 0,1 à 10 en poids et la proportion de charbon actif dans ledit désodorisant est de 10 à 95 % en poids.

4. Désodorisant selon la revendication 1 ou 2, dans lequel la surface spécifique du charbon actif est d'au moins 600 m$^2$/g.

5. Utilisation de la composition de l'une quelconque des revendications 1 à 4 comme désodorisant.

7